# EUROPÄISCHE PATENTSCHRIFT

(11) **EP 0 569 760 B1**
(45) Veröffentlichungstag und Bekanntmachung des Hinweises auf die Patenterteilung: **30.10.1996**
(21) Anmeldenummer: 93106678.1
(22) Anmeldetag: 24.04.1993
(51) Int. Cl.: C07C 49/293, C07C 45/54

(54) **Verfahren zur Herstellung von 1-Cyclopropylbutan-1,3-dion**
Process for the preparation of 1-cyclopropylbutan-1,3-dione
Procédé pour la préparation du cyclopropyl-1-butanedione-1,3

(30) Priorität: 11.05.1992 DE 4215388
(43) Veröffentlichungstag der Anmeldung: 18.11.1993
(73) Patentinhaber: BASF Aktiengesellschaft, 67063 Ludwigshafen (DE)
(72) Erfinder: Northemann, Andreas, Dr., W-6700 Ludwigshafen (DE); Fischer, Martin, Dr., W-6700 Ludwigshafen (DE)

(56) Entgegenhaltungen:
- US-A- 2 395 800
- TETRAHEDRON, Bd.44, Nr.5, 1988, OXFORD GB Seiten 1523 - 1534 RONALD GRIGG ET AL. 'X = Y - ZH systems as potential 1, 3-dipoles. Cyclopropyl substituted azomethine ylides as mechanistic probes in 1, 3 -dipolar cycloaddition reactions'
- INDUSTRIAL AND ENGINEERING CHEMISTRY, Bd.41, Nr.12, Dezember 1949 Seiten 2920 - 2924 HUGH J. HAGEMEYER, JR. ET AL. 'Reactions of isopropenyl acetate'
- Rodd's Chemistry of Carbon Compounds, 2nd Ed., Vol. II, Alicyclic Compounds, Part A, p. 32

## Beschreibung

Die vorliegende Erfindung betrifft ein neues Verfahren zur Herstellung von 1-Cyclopropylbutan-1,3-dion durch thermische Gasphasenreaktion von 1-Cyclopropylvinylacetat.

In US-A 2 395 800 wird allgemein die Herstellung von 1,3-Diketonen durch thermische Umlagerung entsprechender Vinylester beschrieben, wobei als Rest R² auch cycloaliphatische Reste genannt werden. Ausführungen, was unter cycloaliphatischen Resten gemeint sein könnte, sind weder dem allgemeinen Teil der Beschreibung noch den spezifischen Herstellbeispielen zu entnehmen.

Aus Rodd's Chemistry of Carbon Compounds, 2nd edition, Vol. II, Alicyclic Compounds, Part A, Seite 32, vorletzter Absatz ist bekannt, daß Cyclopropan bei Temperaturen von 400 bis 500°C zu Propen umlagert.

Aus J. Org. Chem. 685 bis 692 (1952) ist die Herstellung von 1-Cyclopropylbutan-1,3-dion aus Cyclopropylmethylketon durch Umsetzung mit Essigester in Gegenwart von überstöchiometrischen Mengen an Natriumamid bekannt.

Aus der EP-A-108 708 ist die Herstellung von 1-Cyclopropylbutan-1,3-dion aus Cyclopropancarbonsäurederivaten und Aceton in Gegenwart von Bortrifluorid bekannt.

Diese Verfahren haben den Nachteil, daß dabei große Mengen an Salz anfallen, die abgetrennt werden müssen. Ferner lassen die Ausbeuten zu wünschen übrig, da viele Nebenprodukte auftreten.

Der vorliegenden Erfindung lag also die Aufgabe zugrunde, ein verbessertes Verfahren zur Herstellung von 1-Cyclopropylbutan-1,3-dion zu finden, das den oben genannten Nachteilen abhilft.

Demgemäß wurde ein neues und verbessertes Verfahren zur Herstellung von 1-Cyclopropylbutan-1,3-dion gefunden, welches dadurch gekennzeichnet ist, daß man 1-Cyclopropylvinylacetat Temperaturen von 300 bis 600°C in der Gasphase aussetzt.

Das erfindungsgemäße Verfahren läßt sich wie folgt durchführen:

1-Cyclopropylbutan-1,3-dion läßt sich besonders vorteilhaft herstellen, wenn man das entsprechende Enolacetat flüssig oder gasförmig in einen bei erhöhter Temperatur betriebenen Reaktor eindüst und durch thermische Umlagerung in das genannte Produkt überführt. Solche Reaktoren können z.B. Festbett- aber auch Wirbelschichtreaktoren sein. Da diese mit einem wärmeübertragenden Material füllen oder aber auch als Lehrrohr betreiben.

Als wärmeübertragende Medien kommen alle bei Reaktionstemperatur inerten Materialien wie zum Beispiel Aluminiumoxid, Stähle oder auch keramische Materialien in Frage.

Als Temperaturintervall für die thermische Umlagerung kommt ein Bereich zwischen 300 und 600°C in Frage, bevorzugt ist ein Bereich zwischen 400 und 550°C. Höhere oder niedrigere Temperaturen sind auch möglich, bringen aber keinen Vorteil. Der Reaktionsdruck kann in weiten Bereichen gewählt werden und beträgt in aller Regel 0,01 bis 50 bar, bevorzugt 0,1 bis 5 bar, besonders bevorzugt ist Normaldruck (Atmosphärendruck).

Bei rein thermischen Reaktionen ist die Verweilzeit eng mit der angewandten Reaktionstemperatur verknüpft. Zweckmäßigerweise wählt man Verweilzeiten von 0,1 bis 50 Sekunden, wenn die Reaktionstemperatur im Bereich zwischen 400 und 550°C liegt. Zum Einstellen des bevorzugten Verweilzeitbereichs verwendet man unter Reaktionsbedingungen inerte Gase. Als solche kommen in Betracht Stickstoff, Kohlenoxide, Edelgase, Wasserdampf oder auch Mischungen dieser Gase. Bevorzugtes Gas zum Einstellen der Verweilzeit ist Stickstoff.

Die Ausgangsverbindungen kann man gasförmig durch vorheriges Verdampfen in den Reaktor einleiten oder auch bei Verwendung einer Wirbelschicht flüssig in den auf Reaktionstemperatur befindlichen Reaktor fördern.

Die Raum-Zeit-Ausbeute, hierin definiert als Menge Ausgangsmaterial pro Menge an Wirbelmaterial und Stunde, hat nur einen geringen Einfluß auf die Ausbeute an 1-Cyclopropylbutan-1,3-dion und ist in weiten Grenzen variabel.

Dem Reaktor kann ein Wärmetauscher nachgeschaltet werden, um die flüssigen Reaktionsprodukte zu kondensieren. Die so erhaltenen Reaktionsgemische können durch konventionelle Methoden wie z.B. Destillation oder Rektifikation aufgearbeitet und das Diketon isoliert werden.

Das durch die thermische Umlagerung entstehende Diketon ist; ein wertvoller Synthesebaustein für die Herstellung von pharmazeutischen Präparaten, aber auch für die Herstellung von Pflanzenschutzmitteln wie z.B. aus EP-A-410,726, EP-A-434,624 und EP-A-310,550 bekannt ist. Ferner werden solche Diketone zur Herstellung von Übergangsmetallsalzen verwendet.

### Beispiele

### Beispiel 1

In einem Wirbelschichtreaktor mit einem Innendurchmesser von 10 cm und einer Höhe von 50 cm befinden sich 3,3 kg eines keramischen Materials mit einem Wirbelpunkt von 4,7 cm/s. Der zum Fluidisieren notwendige Stickstoff wird vertikal gegen die Schwerkraft in die Wirbelschicht eingeleitet. Der Wirbelschichtreaktor wird auf 450°C aufgeheizt. Nach Erreichen der Reaktionstemperatur fördert man mittels einer Dosierpumpe 0,5 kg 1-Cyclopropylvinylacetat pro Stunde radial über eine Düse in die Wirbelschicht. Mit Hilfe des Inertgases stellt man eine Verweilzeit von 5 sec ein. Nach einer Vorlaufzeit von 30 min beginnt man, die Reaktionsprodukte im Austragsgefäß zu sammeln. Nach 8 Stunden Laufzeit wurde der Versuch abgebrochen und der gesammelte Austrag von 3,85 kg destillativ aufgearbeitet. Es wurde eine Fraktion von 2,42 kg 1-Cyclopropylvinylacetat erhalten, das sich nicht umgesetzt hat. Eine weitere Fraktion, die 1,37 kg 1-Cyclopropylbutan-1,3-dion enthielt, konnte ebenfalls isoliert werden. Das entspricht einer Ausbeute von 87 % bezogen umgesetztes und 34 % bezogen auf eingesetztes 1-Cyclopropylvinylacetat. Das zurückgewonnene Ausgangsmaterial kann ohne weitere Behandlung wieder eingesetzt werden.

### Beispiel 2

Man verfährt analog Beispiel 1, lediglich die Reaktionstemperatur wird auf 480°C angehoben. Innerhalb von 8 Stunden dosiert man 4 kg in die Wirbelschicht. Am Ende des Versuchs fanden sich 3,78 kg Produktgemisch im Austragsgefäß, die destillativ aufgearbeitet wurden. Es wurde eine Fraktion von 2,03 kg Ausgangsmaterial erhalten. Eine weitere Fraktion, die 1,65 kg 1-Cyclopropyl-1.3-butandion enthält, wurde ebenfalls isoliert; das entspricht einer Ausbeute von 84 % bezogen auf umgesetztes Cyclopropylvinylacetat.

### Beispiel 3

Ein Rohrreaktor mit einem Innedurchmesser von 3 cm und einer Länge von 50 cm, der mit Raschig-Ringen aus Keramik gefüllt und dem ein Verdampfer vorgeschaltet ist, wird auf 450°C aufgeheizt. Bei dieser Temperatur leitet man einen Strom von 200 g/h 1-Cyclopropylvinylacetat so durch den Reaktor, daß eine Verweilzeit von 8 sec erreicht wird. Über einen Zeitraum von 8 Stunden wurden insgesamt 1,6 kg Ausgangsverbindung eingesetzt. Aus den kondensierten 1583 g Produktgemisch konnten destillativ 688 g 1-Cyclopropylvinylacetat (43 %) zurückgewonnen werden, die Ausbeute an 1-Cyclopropylbutan-1,3-dion betrug 793 g, das entspricht 87 % bezogen auf umgesetztes Ausgangsmaterial.

### Beispiele 4 bis 8

In den Beispielen 4 bis 8 wurde analog dem Beispiel 3 verfahren, wobei Reaktionstemperatur und Verweilzeit (VWZ) variiert wurden, als Ausgangsmaterial diente Cyclopropylvinylacetat. Die angegebenen Ausbeuten beziehen sich auf umgesetztes Ausgangsmaterial. Die Reaktionszeit betrug 4 Stunden, wobei jeweils insgesamt 2 kg eingesetzt wurden. Die entsprechenden Daten zu den Versuchen 4 bis 8 sind Tabelle 1 zu entnehmen.

| Beispiel-Nr | 4 | 5 | 6 | 7 | 8 |
|---|---|---|---|---|---|
| Temperatur [°C] | 470 | 495 | 515 | 480 | 480 |
| VWZ [sec] | 10 | 10 | 10 | 12 | 8 |
| Dauer [h] | 4 | 4 | 4 | 4 | 4 |
| Umsatz [%] | 68 | 77 | 92 | 73 | 62 |
| Ausbeute [%] | 83 | 85 | 82 | 91 | 89 |

## Patentansprüche

1. Verfahren zur Herstellung von 1-Cyclopropylbutan-1,3-dion, dadurch gekennzeichnet, daß man 1-Cyclopropylvinylacetat Temperaturen von 300 bis 600°C in der Gasphase aussetzt.

2. Verfahren zur Herstellung von 1-Cyclopropylbutan-1,3-dion nach Anspruch 1, dadurch gekennzeichnet, daß man 1-Cyclopropylvinylacetat Temperaturen von 400 bis 550°C in der Gasphase aussetzt.

3. Verfahren nach Anspruch 2, dadurch gekennzeichnet, daß die Verweilzeit in der Gasphase 0,1 bis 50 Sek. beträgt.

## Claims

1. Process for preparing 1-cyclopropylbutane-1,3-dione, which comprises exposing 1-cyclopropylvinyl acetate to temperatures of from 300 to 600°C in the gas phase.

2. A process for preparing 1-cyclopropylbutane-1,3-dione as claimed in claim 1, wherein 1-cyclopropylvinyl acetate is exposed to temperatures of from 400 to 550°C in the gas phase.

3. A process as claimed in claim 2, wherein the hold-up time in the gas phase is from 0.1 to 50 sec.

## Revendications

1. Procédé de préparation de la 1-cyclopropylbutane-1,3-dione, caractérisé en ce que l'on expose l'acétate de 1-cyclopropylvinyle à des températures de 300 à 600°C en phase gazeuse.

2. Procédé de préparation de la 1-cyclopropylbutane-1,3-dione suivant la revendication 1, caractérisé en ce que l'on expose l'acétate de 1-cyclopropylvinyle à des températures de 400 à 550°C en phase gazeuse.

3. Procédé suivant la revendication 2, caractérisé en ce que la durée de séjour dans la phase gazeuse varie de 0,1 à 50 secondes.
